# EUROPEAN PATENT APPLICATION

(11) **EP 4 494 593 A1**
(43) Date of publication of application: **22.01.2025**
(21) Application number: 23306238.9
(22) Date of filing: 18.07.2023
(51) Int. Cl.: A61B 34/20, A61B 34/10

(54) **DEVICE FOR ASSISTING THE GUIDING OF A THERAPY DELIVERY INSTRUMENT**

(71) Applicant: Fondation de Coopération Scientifique, 67091 Strasbourg Cedex (FR); Institut De Recherche Contre Les Cancers De L'Appareil Digestif, 67091 Strasbourg (FR)
(72) Inventor: BRIDAULT, Flavien, 67700 SAVERNE (FR); COLLINS, Toby, 67000 STRASBOURG (FR); GIMÉNEZ, Mariano Eduardo, 67000 STRASBOURG (FR); HOSTETTLER, Alexandre, 67100 STRASBOURG (FR); VERDE, Juan Manuel, 67000 STRASBOURG (FR)
(74) Representative: Plasseraud IP

(57) **Abstract**

A device for guiding a therapy delivery instrument to a target region of an organ to be treated is proposed, which comprises a processor and a non-transitory computer-readable medium comprising instructions stored thereon which, when executed by the processor cause the device to determine a relative position of the target region with respect to a reference positioning tracker inserted into the organ, determine in a coordinate system relative coordinates of the target region with respect to the reference positioning tracker, determine a relative position of a therapy delivery instrument relatively to the target region.

## Description

The present subject disclosure generally relates to the domain of medical devices, and more specifically to devices and systems configured for guiding tools in the context of non-invasive or at least minimal or minimally invasive surgery.

When performing these interventions, after an operator (for example a surgeon) has identified a region of an organ (e.g., a tumor in a lung or liver) to be treated, (or other therapy to be delivered), the operator may guide tool(s) which will be used for delivering a treatment to the region to be treated.

For these purposes, the operator typically relies on images for guidance of the tools. Such images can be obtained through an endoscope (video cameras), or using medical imaging techniques (for example, computed tomography, a.k.a. CT scans, etc.).

However, using an endoscopic camera result in manipulating an additional tool, which makes the procedure more complex. In addition, endoscopic cameras are useless inside some organs, such as the brain, the liver, the lungs, etc., as they require free space where the region to operate is at a surface of a tissue and not inside the tissues of an organ. As a consequence, the camera cannot see-through the organ.

In addition, because organs tend to move during breathing or due to heartbeat, it is not possible to perform scans or CT scans at a sufficiently high frequency (rate, e.g. expressed in number of scans performed per time unit) in order to keep track of the position of the region to operate. Performing scans or CT scans at a sufficiently high frequency would require taking scans at a frequency higher than 30 scans per second. The radiation produced by the scanner may be harmful, and should therefore be reduced as much as possible (as well as the injected volume of contrast agents used in the scan procedure), which makes the performing scans or CT scans at frequencies higher than 30 scans per seconds undesirable.

As a consequence, the operator will typically rely on a few pre-acquired images, which renders the guidance of the tool(s) more hazardous and inaccurate, in particular in cases where the region to be treated is small and/or deep in the tissue.

Therefore, it is desirable to improve the efficiency of techniques for guiding tools used for delivering a treatment to a region of live tissues, while improving precision and accuracy of such techniques. The present subject disclosure aims at improving the situation.

It is an object of the present subject disclosure to provide an improved device for guiding a therapy delivery instrument to a target region of an organ to be treated.

To achieve these objects and other advantages and in accordance with the purpose of the present subject disclosure, as embodied and broadly described herein, in one aspect of the present subject disclosure, a device configured for guiding a therapy delivery instrument to a target region of an organ (e.g. a human organ) to be treated is proposed, which comprises: a processor; and a non-transitory computer-readable medium comprising instructions stored thereon which, when executed by the processor cause the device to: determine a relative position of the target region with respect to a reference positioning tracker inserted into the organ obtained using a technique for detecting a region to be treated and the reference positioning tracker; determine in a coordinate system relative coordinates of the target region with respect to the reference positioning tracker, wherein the determination of the relative coordinates is based on the relative position of the target region with respect to the reference positioning tracker; determine a relative position of a therapy delivery instrument relatively to the target region based on: the relative coordinates of the target region with respect to the reference positioning tracker; current coordinates in the coordinate system of the therapy delivery instrument obtained using a spatial positioning technique; and current coordinates in the coordinate system of the reference positioning tracker obtained using the spatial positioning technique.

In one or more embodiments, an initialization phase of the proposed device may advantageously be performed, during which relative coordinates of the target region (for example a tumor) with respect to a reference positioning tracker may be determined. Determination of the relative position of the target region with respect to the reference positioning tracker may be performed according to any suitable technique (also referred to herein as the "first technique"). For example, any medical imaging technique usable for detecting both a region to be treated and a reference positioning tracker may be used in some embodiments.

In some embodiments, the first technique may be used in an initialization phase of the proposed device as it is no longer required once the determination of the relative position of the target region with respect to the reference positioning tracker is completed. In particular, in some embodiments, the first technique may not be required for determining the relative position of the therapy delivery instrument relatively to the target region (which may be used to guide the therapy delivery instrument to the target region according to the present subject disclosure).

In some embodiments, once the position of the target region is known with respect to the reference positioning tracker, guidance of tool(s) may be performed using the position of the reference positioning tracker and the position of the therapy delivery instrument, which positions may be obtained using another technique (also referred to herein as the "second technique"). As a consequence, in some embodiments, once the first technique has been used for obtaining the position of the target region relative to the reference positioning tracker, it may no longer be used for guidance of the tools, which instead may use the second technique.

In one or more embodiments, the second technique may comprise any suitable spatial positioning technique (such as, for example, a technique using the determining of position(s) of one or more electromagnetic sensors in an electromagnetic field). Any spatial positioning technique can be used as a second technique, preferably one that is not harmful for the patient and/or the operator, and/or that can track position(s) over time with great precision.

Therefore, advantageously, the proposed device can be used for the assistance or guidance (of a therapy delivery instrument to a target region of live tissues (e.g. an organ)) with a greater precision while diminishing the patient's exposure to harmful radiation, reducing the necessity of contrast agent and limiting unnecessary damage to the target organ, without requiring using the medical imaging technique which was used to detect anomalies which define the target region.

In addition, advantageously, the reference positioning tracker may be inserted in the organ to be treated, and may follow the movement of this organ. Therefore, the reference positioning tracker may advantageously be subjected to movements that are (substantially) similar to that of the target region, which allows accurately following such movements through the reference positioning tracker.

Therefore, as in some embodiments the proposed device may be configured for determining the relative position of the therapy delivery instrument relatively to the target region based on: current coordinates of the reference positioning tracker, and relative coordinates of the target region with respect to the reference positioning tracker, the proposed device may be able to operate in consideration of the movement of the target region through time. As a consequence, the proposed device may advantageously be able to track (monitor) changes over time of the position of the therapy delivery instrument relatively to the actual position of the target region, instead of determining a potentially outdated position of the target region.

Advantageously, the proposed device allows determining the position of a therapy delivery instrument relatively to a target region with great accuracy, including in situations where the position of the target region moves over time (e.g. during breathing of the patient or due to the heartbeat of the patient), which allows for example guiding a therapy delivery instrument with improved efficiency and accuracy.

In one or more embodiments, information on the position of the therapy delivery instrument relatively to the target region determined by the proposed device may be provided to an operator, for example through displaying it on a screen (or a display). In some embodiments, information on the position of the therapy delivery instrument relatively to the target region determined by the proposed device may be used to instruct a (medical) robot, e.g. configured for guiding the therapy delivery instrument to the target region.

In one or more embodiments, the reference positioning tracker may be a reference needle.

A reference needle may advantageously be used as a reference positioning tracker in order to reduce the movements of the organ and/or to constrain its motion or degrees-of-freedom, as the needle is typically inserted in the organ and at least the skin of the patient. In embodiments in the context of minimally invasive therapies, the different tools used for the procedure or for treating the target region of the organ may not be inserted in the organ through large incisions but through adjusted holes. As a consequence, their movements may be reduced in directions parallel to the skin due to the skin and underlying soft tissue(s). Therefore, the tracking of the reference positioning tracker is advantageously rendered more predictable, while uncertainties are reduced. As a result, tracking is eased, and the precision achieved for guiding the tool(s) is enhanced.

In one or more embodiments, the proposed device may be further configured for determining the current coordinates of the reference positioning tracker using a first electromagnetic sensor configured for producing signals in response to an electromagnetic field produced by an electromagnetic generator. In some embodiments, the electromagnetic sensor may be related to the reference positioning tracker. In some embodiments, the proposed device may be further configured for determining the current coordinates of the therapy delivery instrument based on signals received from at least a second electromagnetic sensor in response to the electromagnetic field, with the second electromagnetic sensor being related to the therapy delivery instrument.

In some embodiments, the spatial positioning technique used to determine the current coordinates of the reference positioning tracker and the current coordinates of the therapy delivery instrument may be based on electromagnetic sensors which are placed respectively on the reference positioning tracker and the therapy delivery instrument. In such embodiments, information on the positions of the tracker and the instrument can advantageously be obtained through information on one or more of the positions and orientations of the sensors. The positions and/or orientations of the electromagnetic sensors may be obtained by the current intensity produced in the coils of the sensors when immersed in the magnetic field produced by the electromagnetic generator. This advantageously enables tracking over time the potentially changing positions and orientations of the each or both of the sensors, which reflect (are linked to)(correspond to) the potentially changing positions of each or both of the reference positioning tracker and the therapy delivery instrument.

In one or more embodiments, a sensor may be related to the reference positioning tracker through a mechanical linkage associating the sensor and the reference positioning tracker. Likewise, in one or more embodiments, a sensor may be related to the therapy delivery instrument through a mechanical linkage associating the sensor and the therapy delivery instrument. Depending on the embodiment, such mechanical linkage may be of any suitable type which provides information on the position of the tracker (or, as the case may be, the instrument). For example, one or more sensors may be fixedly coupled (e.g. fixed) on one or more of the therapy delivery instrument and the reference positioning tracker, so that the position of the therapy delivery instrument or the reference positioning tracker (as applicable) can be determined in real-time or almost real-time based on the position of the sensor to which it is (fixedly) coupled (linked)(fixed). As another example, one or more sensors may be rigidly fixed on one or more of the therapy delivery instrument and the reference positioning tracker. In yet another example, a sensor may be in a slide link relationship with the tracker and/or the instrument. In that case, the position of the instrument and/or the tracker is determined to be on a line.

In one or more embodiments, the device may further comprise a guiding device configured to guide the therapy delivery instrument. The guiding device may comprise a third electromagnetic sensor configured for producing signals in response to the electromagnetic field. In some embodiments, the second electromagnetic sensor may be integral with the therapy delivery instrument. In some embodiments, the proposed device may be further configured for determining the current coordinates of the therapy delivery instrument based on signals received from the third electromagnetic sensor in response to the electromagnetic field.

In some embodiments, the second electromagnetic sensor may be fixed to the therapy delivery instrument. Depending on the embodiment, the second electromagnetic sensor may be fixed at any position on the therapy delivery instrument. The third electromagnetic sensor may advantageously be used to determine the position of the guiding device, in particular the axis given by the guiding device to the therapy delivery instrument, while the second electromagnetic sensor may advantageously be used to track the movement (or, depending on the embodiment, track changes over time of the position) of the therapy delivery instrument in the guide (for example, the depth of insertion of the therapy delivery instrument in the guiding device which correlates with the depth of insertion of the therapy delivery needle).

For example, in one or more embodiments, the proposed device may be further configured for determining the relative position of the therapy delivery instrument relatively to the target region may be based: on angular coordinates of therapy delivery instrument obtained based on signals received from the third electromagnetic sensor, and/or on insertion depth value of the therapy delivery instrument relatively to the guiding device obtained based on signals received from the second and third electromagnetic sensors.

Advantageously, a more accurate position of the therapy delivery instrument can be obtained especially if the sensor is placed on the working end of the therapy delivery instrument, and, depending on the embodiment, the sensor can be positioned at any point of the therapy delivery instrument with very few modifications of the instrument.

In one or more embodiments, the second electromagnetic sensor may be positioned in a front part of the therapy delivery instrument.

Advantageously, an accurate position of the working end of the therapy delivery instrument may be obtained. Depending on the embodiment, the working end or front part of the therapy delivery instrument may comprise a part of the therapy delivery instrument which delivers the therapy to the organ (in some embodiments specifically to the target region of the organ). For example, the front part of the therapy delivery instrument may be the tip end of the therapy delivery instrument.

In one or more embodiments, the proposed device may be further configured for determining the current coordinates of the reference positioning tracker based on information related to a current position of a first arm of a medical robot and the current coordinates of the therapy delivery instrument are determined based on information related to a current position of a second arm of the medical robot.

Advantageously, a position of the therapy delivery instrument (in particular of its working end) and/or of the reference positioning tracker with improved accuracy can be obtained without requiring specific modifications of the instrument and tracker.

In one or more embodiments, the proposed device is configured to be used with a (medical) robot. In some embodiments, the proposed device may be operatively coupled with a (medical) robot and may further be configured for determining current coordinates of the reference positioning tracker based on information on a current position of a first arm of the robot. In some embodiments, the proposed device may be further configured to determine current coordinates of the therapy delivery instrument based on information on a current position of an electromagnetic sensor at any part of the therapy delivery instrument.

In one or more embodiments, the proposed device is configured to be used with a (medical) robot. In some embodiments, the proposed device may be operatively coupled with a (medical) robot and may further be configured for determining current coordinates of the reference positioning tracker based on information of an electromagnetic sensor positioned at a part of the reference positioning tracker. In some embodiments, the proposed device may further be configured for determining current coordinates of the therapy delivery instrument based respectively on information on a current position of an arm of the medical robot, and an electromagnetic sensor positioned at any point of the medical robot.

In one or more embodiments, the proposed device may further be configured to use, as the technique used for detecting a region to be treated, a medical imaging technique. In such embodiments, the proposed device may further be configured to use an image obtained by the medical imaging technique for determining the relative position of the target region with respect to a reference positioning tracker inserted into the organ.

This advantageously allows obtaining an accurate position of the target region relatively to the reference positioning tracker.

In one or more embodiments, the coordinate system is a frame of reference centered on a point of the reference positioning tracker and with static axes relatively to the reference positioning tracker.

In such embodiments, the origin of the coordinate system used has its origin attached to the reference positioning tracker and the axes are fixed relatively to the reference positioning tracker. Therefore, the reference positioning tracker is fixed in the frame of reference (that is the coordinate system), which advantageously allows reducing the computing required to determine the relative position of a therapy delivery instrument relatively to the target region, since the movement of the organ is automatically taken into consideration when considering the position of the reference positioning tracker without adding computing (which would be the case for example if the frame of reference was fixed to the operating room).

In one or more embodiments, the non-transitory computer-readable medium may comprise further instructions stored thereon which, when executed by the processor cause the device to determine a deformation parameter of the organ, wherein determining the relative position of the therapy delivery instrument relatively to the target region is based on the deformation parameter of the organ.

In cases where the movement of the organ (which is tracked by the reference positioning tracker) deforms the organ, the relative position of the target region with respect to the reference positioning tracker may be affected through time. Therefore, taking into consideration the deformation parameter advantageously allows determining a more accurate relative position of a therapy delivery instrument relatively to the target region.

For example, it is possible to determine updated relative coordinates of the target region with respect to the reference positioning tracker according to the deformation parameter and to use these updated relative coordinates of the target region with respect to the reference positioning tracker to determine the relative position of the therapy delivery instrument relatively to the target region.

In one or more embodiments, the deformation parameter may comprise any parameter related to the deformation of the organ, for example the elasticity, density, viscosity or ductility of the organ. This deformation parameter can be known or can be obtained for example when inserting the reference positioning tracker, therefore, adapting the deformation parameter to each individual.

In one or more embodiments, the proposed device is operatively coupled with a (medical) robot, and the non-transitory computer-readable medium may comprise further instructions stored thereon which, when executed by the processor cause the device to send an order to the (medical) robot to reduce a distance between the therapy delivery instrument and the target region based on the relative position of the therapy delivery instrument relatively to the target region.

In another aspect of the present subject disclosure, a computer program product is provided, which comprises code instructions that, when said instructions are run by at least a processor, instruct the processor to perform a method comprising: determining a relative position of the target region with respect to a reference positioning tracker inserted into the organ obtained using a technique for detecting a region to be treated and the reference positioning tracker; determining in a coordinate system relative coordinates of the target region with respect to the coordinates of the reference positioning tracker, said determination of the relative coordinates being based on the relative position of the target region with respect to the reference positioning tracker; determining a relative position of a therapy delivery instrument relatively to the target region based on: the relative coordinates of the target region with respect to the reference positioning tracker; current coordinates in the coordinate system of the therapy delivery instrument obtained using a spatial positioning technique; and current coordinates in the coordinate system of the reference positioning tracker obtained using the spatial positioning technique.

In yet another aspect of the present subject disclosure, a non-transitory computer-readable medium encoded with executable instructions which, when executed, causes an apparatus comprising a processor operatively coupled with a memory, to perform a method comprising: determining a relative position of the target region with respect to a reference positioning tracker inserted into the organ obtained using a technique for detecting a region to be treated and the reference positioning tracker; determining in a coordinate system relative coordinates of the target region with respect to the coordinates of the reference positioning tracker, said determination of the relative coordinates being based on the relative position of the target region with respect to the reference positioning tracker; determining a relative position of a therapy delivery instrument relatively to the target region based on: the relative coordinates of the target region with respect to the reference positioning tracker; current coordinates in the coordinate system of the therapy delivery instrument obtained using a spatial positioning technique; and current coordinates in the coordinate system of the reference positioning tracker obtained using the spatial positioning technique, is proposed.

In yet another aspect of the present subject disclosure, a computer program product comprising computer program code tangibly embodied in a computer readable medium, said computer program code comprising instructions to, when provided to a computer system and executed, cause said computer to perform a method comprising: determining a relative position of the target region with respect to a reference positioning tracker inserted into the organ obtained using a technique for detecting a region to be treated and the reference positioning tracker; determining in a coordinate system relative coordinates of the target region with respect to the coordinates of the reference positioning tracker, said determination of the relative coordinates being based on the relative position of the target region with respect to the reference positioning tracker; determining a relative position of a therapy delivery instrument relatively to the target region based on: the relative coordinates of the target region with respect to the reference positioning tracker; current coordinates in the coordinate system of the therapy delivery instrument obtained using a spatial positioning technique; and current coordinates in the coordinate system of the reference positioning tracker obtained using the spatial positioning technique, is proposed.

In another aspect of the present subject disclosure, a data set representing, for example through compression or encoding, a computer program as proposed herein, is proposed.

It should be appreciated that the present invention can be implemented and utilized in numerous ways, including without limitation as a process, an apparatus, a system, a device, and as a method for applications now known and later developed. These and other unique features of the system disclosed herein will become more readily apparent from the following description and the accompanying drawings. The present subject disclosure is illustrated by way of example, and not by way of limitations, in the figures of the accompanying drawings, in which like reference numerals refer to similar elements and in which:
- Figure 1 illustrates a system using a spatial positioning technique based on electromagnetic sensors and an electromagnetic field in accordance with one or more embodiments.
- Figure 2 illustrates a system using a spatial positioning technique based on a position of an arm of a medical robot in accordance with one or more embodiments.
- Figure 3 illustrates the determination of a relative position of a therapy delivery instrument relatively to a target region in accordance with one or more embodiments.
- Figure 4 illustrates a flowchart representing an implementation of a system in accordance with one or more embodiments.

For simplicity and clarity of illustration, the drawing figures illustrate the general manner of construction, and descriptions and details of well-known features and techniques may be omitted to avoid unnecessarily obscuring the discussion of the described embodiments of the invention. Additionally, elements in the drawing figures are not necessarily drawn to scale. For example, the dimensions of some of the elements in the figures may be exaggerated relative to other elements to help improve understanding of embodiments of the present invention. Certain figures may be shown in an idealized fashion in order to aid understanding, such as when structures are shown having straight lines, sharp angles, and/or parallel planes or the like that under real-world conditions would likely be significantly less symmetric and orderly. The same reference numerals in different figures denote the same elements, while similar reference numerals may, but do not necessarily, denote similar elements.

In addition, it should be apparent that the teaching herein can be embodied in a wide variety of forms and that any specific structure and/or function disclosed herein is merely representative. In particular, one skilled in the art will appreciate that an aspect disclosed herein can be implemented independently of any other aspects and that several aspects can be combined in various ways.

The present disclosure is described below with reference to functions, engines, block diagrams and flowchart illustrations of the methods, systems, and computer program according to one or more exemplary embodiments. Each described function, engine, block of the block diagrams and flowchart illustrations can be implemented in hardware, software, firmware, middleware, microcode, or any suitable combination thereof. If implemented in software, the functions, engines, blocks of the block diagrams and/or flowchart illustrations can be implemented by computer program instructions or software code, which may be stored or transmitted over a computer-readable medium, or loaded onto a general purpose computer, special purpose computer or other programmable data processing apparatus to produce a machine, such that the computer program instructions or software code which execute on the computer or other programmable data processing apparatus, create the means for implementing the functions described herein.

Embodiments of computer-readable media include, but are not limited to, both computer storage media and communication media including any medium that facilitates transfer of a computer program from one place to another. As used herein, a "computer storage media" may be any physical media that can be accessed by a computer or a processor. In addition, the terms « memory » and « computer storage media" include any type of data storage device, such as, without limitation, a hard drive, a flash drive or other flash memory devices (e.g. memory keys, memory sticks, key drive), CD-ROM or other optical storage, DVD, magnetic disk storage or other magnetic storage devices, memory chip(s), Random Access Memory (RAM), Read-Only-Memory (ROM), Electrically-erasable programmable read-only memory (EEPROM), smart cards, or any other suitable medium that can be used to carry or store program code in the form of instructions or data structures which can be read by a computer processor, or a combination thereof. Also, various forms of computer-readable media may transmit or carry instructions to a computer, including a router, gateway, server, or other transmission device, wired (coaxial cable, fiber, twisted pair, DSL cable) or wireless (infrared, radio, cellular, microwave). The instructions may comprise code from any computer-programming language, including, but not limited to, assembly, C, C++, Python, Visual Basic, SQL, PHP, and JAVA.

Unless specifically stated otherwise, it will be appreciated that throughout the following description discussions utilizing terms such as processing, computing, calculating, determining, or the like, refer to the action or processes of a computer or computing system, or similar electronic computing device, that manipulate or transform data represented as physical, such as electronic, quantities within the registers or memories of the computing system into other data similarly represented as physical quantities within the memories, registers or other such information storage, transmission or display devices of the computing system.

The terms "comprise," "include," "have," and any variations thereof, are intended to cover a non-exclusive inclusion, such that a process, method, article, or apparatus that comprises a list of elements is not necessarily limited to those elements, but may include other elements not expressly listed or inherent to such process, method, article, or apparatus.

Additionally, the word "exemplary" is used herein to mean "serving as an example, instance, or illustration". Any embodiment or design described herein as "exemplary" is not necessarily to be construed as preferred or advantageous over other embodiments or designs.

In the following description and claims, the terms "coupled" and "connected", along with their derivatives, may be indifferently used to indicate that two or more elements are in direct physical or electrical contact with each other, or two or more elements are not in direct contact with each other, but yet still co-operate or interact with each other.

As used herein, the term "organ" means any organ of the human or animal body. For example, the organ can be a liver, a lung, a brain, a pancreas, a kidney, and retroperitoneal organs.

In one or more embodiments, determining a relative position of the target region with respect to a reference positioning tracker inserted into the organ obtained, using the technique for detecting a region to be treated and the reference positioning tracker may comprise determining the position of the target region with respect to the reference positioning tracker. This determination may be based on a technique for detecting a region to be treated and the reference positioning tracker. That is, the relative position may be determined according to a data set obtained using this technique. The data set may depend on the technique used, and therefore the relative position may also be represented according to the technique used. For example, when the first technique is for example a medical imaging technique a vector may be determined on the image obtained, the vector may be supplemented by data related to (e.g. data which defines) the section which represents the image, and possibly the scale of the image. More generally, the data set may be used to determine data corresponding or at least equivalent to a distance and a direction from the reference positioning tracker to the target region. This corresponding data may be considered as the relative position of the target region with respect to the reference positioning tracker. For example, the combination of the vector completed by the section and the scale of the image fully defines the distance between the reference positioning tracker to the target region and the direction from the reference positioning tracker to the target region.

In one or more embodiments, coordinates of the target region, coordinates of the reference positioning tracker or coordinates of the therapy delivery instrument may comprise any data (for example, tuple of values) which represent (or code for), in a coordinate system, the position of respectively the target region, the reference positioning tracker or the therapy delivery instrument. The coordinates of the reference positioning tracker and the therapy delivery instrument may correspond to the data or signals received from the spatial positioning system which implements the spatial positioning technique.

Depending on the embodiment, the coordinates may be of any type, for example, polar coordinates, cartesian coordinates. As another example, in some embodiments, in the case of a spatial positioning technique using electromagnetic sensors in an electromagnetic field, these coordinates may be represented directly into current intensities generated in the sensors when positioned in the electromagnetic field.

Current coordinates of the therapy delivery instrument and the reference positioning tracker may comprise coordinates of, respectively, the therapy delivery instrument and the reference positioning tracker obtained using the spatial positioning technique shortly (for example, between 1 millisecond to 10 milliseconds) before determining the relative position of the therapy delivery instrument relatively to the target region.

In one or more embodiments, relative coordinates of a first point (in space) with respect to a second point may comprise coordinates based on which, possibly combined with the coordinates of the second point, the coordinates of the first point can be retrieved. Such relative coordinates may be for example a vector. Relative coordinates of a first region with respect to a second region may be equivalent to relative coordinates of a center of the first region with respect to the center of the second region. The center of a region may be determined (computed) with any known manner.

In one or more embodiments, a position of the target region may be determined in respect to the reference positioning tracker in the coordinate system, according to the spatial positioning technique, for determining relative coordinates of the target region with respect to the reference positioning tracker. In some embodiments, a relative position of the target region may be determined in respect to the reference positioning tracker in the same coordinate system used for the coordinates of the reference positioning tracker and the coordinates of the therapy delivery instrument. The determination of the relative coordinates may be based on the relative position of the target region with respect to the reference positioning tracker. For example, in some embodiments, a relative position of the target region in respect to the reference positioning tracker obtained using the first technique may be transformed into relative coordinates in the same coordinate system as that of the coordinates of the reference positioning tracker and the therapy delivery instrument. For example, in some embodiments, data corresponding or at least equivalent to a distance and direction from the reference positioning tracker to the target region, may be transformed into the relative coordinates which corresponds to the same distance and direction, but in the coordinate system used to represent the coordinates of the reference positioning tracker and the therapy delivery instrument.

In one or more embodiments, the spatial positioning technique may comprise a technique that enables to position an element in space, more specifically in a volume containing the organ or at least the target region and its vicinity. For example, the spatial positioning technique may in some embodiments use electromagnetic sensors and an electromagnetic field, wherein the position of the sensor in the electromagnetic field and therefore the position of the sensor in the space can be determined based on the intensity of the current generated by a sensor in the electromagnetic field. In some embodiments, the spatial positioning technique may also use parameters of a (medical) robot, for example, the position of each part of a (medical) robot's arm may be used to retrieve the position in space of the therapy delivery instrument or the reference positioning tracker attached at its end.

In one or more embodiments, a technique for detecting a region to be treated and the reference positioning tracker may comprise any known technique suitable therefor, in particular any technique in the medical field suitable therefor. For example, any suitable medical imaging technique usable for detecting both the region to be treated and the reference positioning tracker may be used depending on the embodiment, such as, for example, a CT scan, magnetic resonance technique, cone-beam CT scans, PET CT scans, 2.5 and/or 3D ultrasound technique, multiple fluoroscopic technique. In some embodiments, the technique for detecting a region to be treated and the reference positioning tracker may use one or more sub-techniques. For example, in some embodiments, the technique may comprise two sub-techniques, such as, for example, a medical imaging technique for detecting the region to be treated (for example an MRI) and a medical imaging technique for detecting the reference positioning tracker (for example a scan). In such case, another organ (for example an artery) or part of the organ may be used as a common reference detected by both sub techniques to enable to cross the information obtained through one sub-technique with the information obtained through the other sub-technique.

In one or more embodiments, detecting an object (for example, a region or a reference positioning tracker) may comprise identifying this object in the organ. Depending on the embodiment, this detection may be used to locate the object in space or may be used to locate the object relatively to another object.

In one or more embodiments, a therapy delivery instrument (or tool) may comprise any instrument used in a medical intervention and/or operation (a surgery), especially any surgical instrument used for non-invasive or mini/minimally invasive surgery. The therapy delivery instrument may in particular be an instrument whose precise positions cannot be determined easily during the operation, for example, an instrument inserted in an organ of the human body during a non-invasive or minimally invasive surgery. Such an instrument may for example be a therapy needle (for example, biopsy needle, ablation needle, drainage needle, injection or infusion needle, etc.), a grasping device, a cutting device, a suturing device. The therapy delivery instrument may also be an ablation electrode, a biopsy needle, a trocar needle, etc... More generally, the therapy delivery instrument may be usable to treat a target region of an organ, for example, by burning or emitting electric pulses in a tumor, or by enabling to perform a biopsy of part of the target region, or by injection of solutions, drainage of liquid, deploying markers, etc..

In one or more embodiments, a target region of an organ may comprise any region of the organ determined as a target for the therapy delivery instrument. The target region may be for example a tumor and the vicinity of the tumor.

In one or more embodiments, a reference positioning tracker may comprise any device the position of which can be determined by the spatial positioning technique, or the position of which can be detected by the first technique. For example, depending on the embodiment, the reference positioning tracker may be a needle or a capsule with one or several electromagnetic sensors, or a needle attached to a medical robot's arm, or any combination of the previously mentioned devices.

In one or more embodiments, the reference positioning tracker may be positioned on the organ by being inserted into the organ. For example, the reference positioning tracker may be implanted in the organ to follow the movement of the organ, for example, organ movements related to respiration and heart activity. For example, the reference positioning tracker may be configured to adhere to the organ when positioned on the organ.

In one or more embodiments, guiding a therapy delivery instrument to a target region may comprise: determining the position of the therapy delivery instrument relatively to the target region, and therefore assisting the guidance of the therapy delivery instrument to the target region.

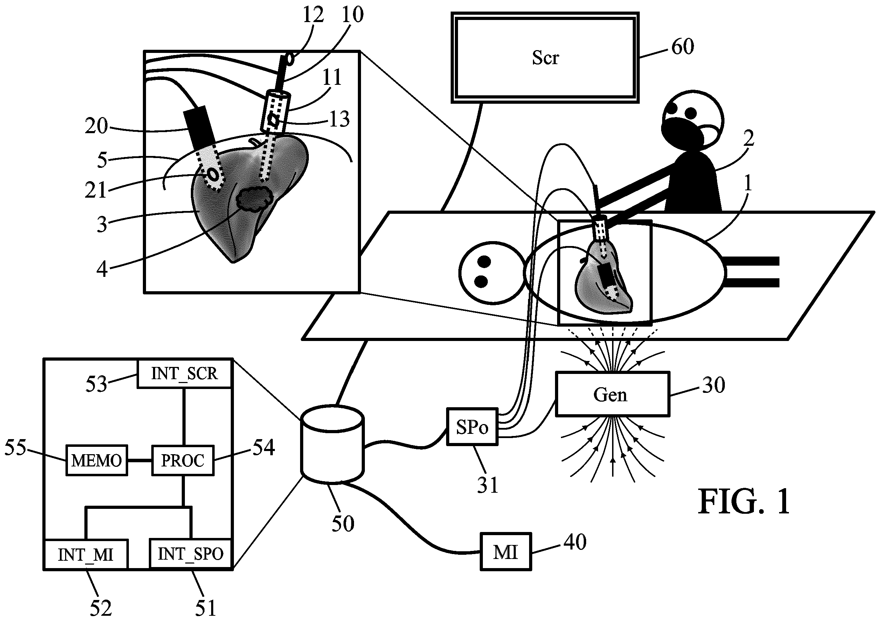

Referring to figure 1, there is shown a patient 1 and an operator 2. The operator 2 is performing a procedure to treat a region of an organ (in the example of Fig. 1 the liver 3). The treatment applied to the region 4 of the liver 3 aims at destroying a tumor 4, this tumor 4 is referred to as the target region 4. To destroy the tumor 4, the operator 2 inserts a therapy delivery instrument 10 in the liver 3. In the example of figure 1, this therapy delivery instrument 10 may be a therapy delivery needle 10, delivering a thermotherapy, that is, the temperature of the tip of the needle 10 may be increased in order to destroy tumor's cells around it.

In order to deliver an optimal or near-optimal therapy, it is desirable for the operator 2 to position the therapy delivery instrument 10 in a specific position, such as, for example, at the center of the tumor 4. The device 50 is configured to assist the operator 2 in determining the position of the therapy delivery instrument 10 in the organ 3, thereby assisting the operator 2 in placing the therapy delivery instrument 10 at an optimal or near-optimal position.

A reference positioning tracker 20 may be inserted through the skin 5 of the patient in the organ 3 before inserting the therapy delivery instrument 10. In the example of figure 1, this reference positioning tracker 20 is a needle 20 comprising a tracker 21. The tracker 21 may be used to determine the position of the needle 20 in the liver 3. In the example of figure 1, the tracker 20 comprises an electromagnetic sensor 20, that is, a sensor composed of one or more coils. The position and/or orientation of the sensor may be determined based on the intensity of the current induced in each coil when the sensor is immersed in the magnetic field generated by the electromagnetic field generator 30.

In one or more embodiments, the therapy delivery instrument 10 is inserted in the organ 3 through the skin 5 of the patient 1 once the reference positioning tracker 20 is positioned. In order to determine the position of the therapy delivery instrument 10, and more specifically the position of the part of the instrument 10 which delivers the therapy (in the case of figure 1 the tip point of the needle 10), at least one tracker 12 may be attached to (or included in) the therapy delivery instrument 10. The exemplary tracker 12 may be an electromagnetic sensor 12, which may in some embodiments be similar to the one comprised in the reference positioning tracker 20. The sensor 12 may be placed near the tip point of the therapy needle 10 to have an accurate position of the tip point of the therapy needle 10. In the example of figure 1, the sensor 12 is not placed at the tip point of the therapy needle 10 but further back from this tip point. However, placing the sensor 12 at the back of the therapy needle 10 may reduce the accuracy of the determination of the position of tip point of the therapy delivery instrument 10 but in such position the sensor can be attached to the instrument without requiring modifications of existing therapy delivery instrument 10. Additional sensors may be added (for example, sensor on the skin of the patient or endocanalicular sensor) in some embodiments in order to mitigate this potential reduction in accuracy. In the example of figure 1, the guiding device 11 (for example a trocar), which is used to orient the insertion of the therapy needle 10 through the skin 5 of the patient and through the organ 3, may also include a tracker 13, which in the case of figure 1 may also be an electromagnetic sensor 12 (e.g. similar to the one comprised in the reference positioning tracker 20).

In one or more embodiments, the signals received from the sensor 13 may be used by the spatial positioning unit 31 to determine the orientation of the guiding device 11. Further, the signals received from the sensor 12 may be used by the spatial positioning unit 31 to determine the insertion depth of the therapy needle 10 with respect to the guiding device 11, and in some embodiments more specifically with respect to the sensor 13 of the guiding device 11.

Therefore, in some embodiments, the combination of information on the insertion depth of the therapy needle 10 and information on the orientation of the guiding device 11 may be used to retrieve an accurate position of the tip point of the therapy needle 10.

In some embodiments, the trackers 12, 13, 21 may comprise electromagnetic sensors, which comprise one or more coils. The position and/or orientation of each sensor may be determined based on the intensity of the current induced in each coil when the sensor is immersed in the magnetic field generated by the electromagnetic field generator 30. The more intense the electromagnetic field (which varies depending on the distance from the generator), the more parallel the axes of the coils to the electromagnetic field lines, and the more intense the current in the coils. The trackers 12, 13, 21 and the electromagnetic field generator 30 may therefore emit signals received by the spatial positioning unit (SPo) 31 based on which the Spo 31 may determine the positions and/or orientations of each electromagnetic sensor 12, 13, 21.

In some embodiments, the device 50 may comprise a spatial positioning interface (INT_Spo) 51 configured to receive the positions and/or orientations determined by the Spo 31.

In some embodiments, the device 50 may further comprise an imaging interface (INT_MI) 52 configured to receive images from a medical imaging unit 40. Such medical imaging (MI) unit 40 may be used to detect both the tumor 4 and the reference positioning tracker 20. Such medical imaging unit 40 may be for example a scanner.

In some embodiments, the device 50 may further comprise a screen interface (INT_SCR) 53 configured to send to a screen (or display) 60 data associated to the relative position of the therapy delivery instrument 10 relatively to the tumor 4.

In some embodiments, the device 50 may further comprise a processing module 54 and a memory unit 55. The memory unit 55 may comprise a non-volatile unit which retrieves the computer program and a volatile unit which retrieves and stores data including one or more of the medical image received from the MI unit 40, the positions and/or orientations of the electromagnetic sensors 12, 13, 21, a vector representing relative coordinates of the tumor 4 with respect to the reference positioning needle 20, a deformation parameter of the organ, updated relative coordinates of the tumor 4 with respect to the reference needle 20, relative positions of a therapy delivery needle 10, etc.

In one or more embodiments, the processing module 54 of the device 50 may be configured to determine one or more of: a vector representing relative coordinates of the tumor 4 with respect to the reference needle 20 based on the relative position of the tumor 4 with respect to the reference needle 20 which is determined according to the medical image; updated relative coordinates of the tumor 4 with respect to the reference needle 20 according to the deformation parameter of the organ; relative positions of a therapy delivery needle 10 relatively to the reference needle 20.

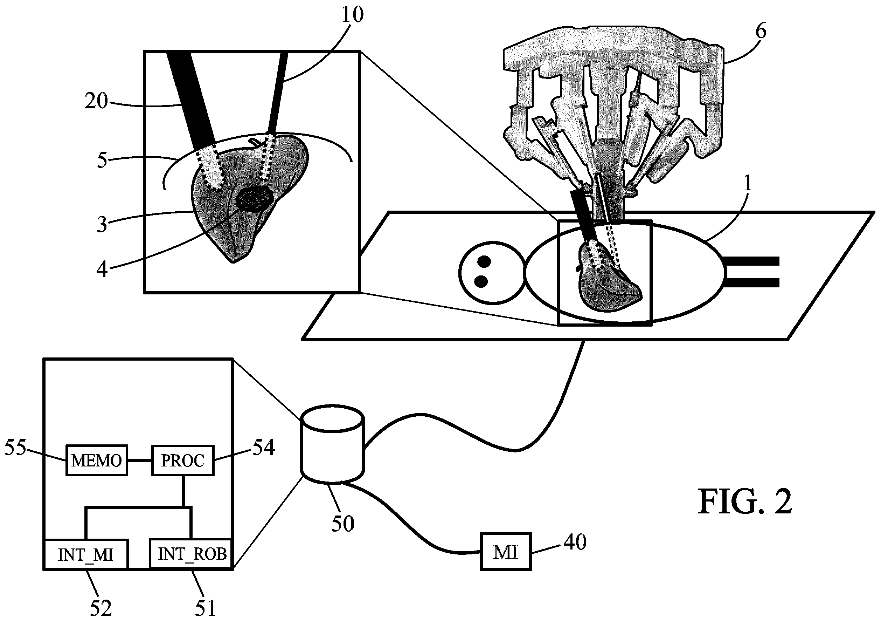

Figure 2 illustrates a system using a spatial positioning technique based on a position of an arm of a medical robot in accordance with one or more embodiments.

As shown on Fig. 2, the medical procedure may be performed by a (medical) robot 6 equipped with one or more arms, instead of a human operator 2 as shown on Fig. 1.

In one or more embodiments, the medical robot 6 may comprise at least two arms. Each of the therapy delivery instrument 10, which in the example of figure 2 is the same therapy delivery needle 10 as in figure 1, and of the reference positioning tracker 20, which in the example of figure 2 is a needle 10 as in figure 1, may be attached to a respective arm of the medical robot 6.

In the embodiment of figure 2, the therapy delivery needle 10 and the reference needle 20 do not need trackers to determine their positions and orientation since they are fixedly coupled (e.g. fixed, for example rigidly fixed with respect to the robot's coordinate system) on different arms of the medical robot, which are associated through a mechanical linkage, and therefore no tracker is represented in figure 2.

In the exemplary case illustrated by figure 2, the medical robot 6 is tasked with positioning the therapy delivery needle 10 in a specific position (e.g. at the center of the tumor 4) to deliver an optimal (or near-optimal) therapy to the patient 1. In some embodiments, the device 50 may be configured to control the medical robot 6 with respect to determining how to move the arm on which the therapy delivery needle 10 is attached in order to place the therapy delivery needle 10 at the optimal (or near-optimal) position (e.g., at the center of the tumor 4).

In some embodiments, the medical robot 6 may be configured to insert the reference positioning tracker 20 (that is, the reference needle 20) through the skin 5 of the patient 1 in the organ 3, preferably prior to inserting the therapy delivery needle 10. To determine the position and/or orientation of the needle 20 in the liver 3 of the patient 1, the medical robot 6 may be configured to determine one or more respective positions of one or more components of the arm on which the needle 20 is fixedly coupled (e.g. fixed, for example rigidly fixed).

In some embodiments, the therapy delivery needle 10 is inserted in the organ 3 through the skin 5 of the patient 1 once the reference positioning needle 20 is positioned. To determine the position and/or orientation of the therapy delivery needle 10 (and more specifically the position of the tip point of the therapy delivery needle 10) in the liver 3 of the patient 1, the medical robot 6 may be configured to determine one or more respective positions of one or more components of the arm on which the therapy delivery needle 10 is fixedly coupled (e.g. fixed, for example rigidly fixed).

It will be appreciated by those having ordinary skill in the relevant art that other techniques for determining the relative positions of the therapy delivery needle 10 and the reference needle 20 may be used in place of or in addition to techniques using positioning information to the robot arm to which the needle is coupled which is given by way of example only according to embodiments of the present subject disclosure.

In one or more embodiments, the medical robot 6 may be configured to determine information on positions and/or orientations in space of the therapy delivery needle 10 and of the reference needle 20 based on positions of one or more components of one or more of its arms, and to send data corresponding to such information to the device 50.

In one or more embodiments, the device 50 may comprise a robot interface (INT_ROB) 56 configured to receive, from the robot 6, data related to positions and/or orientations of the therapy delivery needle 10 and of the reference needle 20, which in some embodiments may have been determined by the robot 6.

Alternatively, in some embodiments, the device 50 may be configured to receive data related to one or more positions of one or more components of one or more of the arms of the medical robot 6, and to determine positions and/or orientations in space of the therapy delivery needle 10 and of the reference needle 20 based on the received data. The robot interface (INT_ROB) 56 may also be configured to send instructions (e.g. control messages) to the medical robot 6.

In one or more embodiments, the device 50 may further comprise an imaging interface (INT_MI) 52 configured to receive images from a medical imaging unit 40. Such medical imaging (MI) unit 40 may be used to detect both the tumor 4 and the reference positioning tracker 20. Such medical imaging unit 40 may be for example a scanner.

In the embodiment of figure 2, the volatile memory unit may be configured for retrieving and storing data comprising the medical image received from the MI unit 40, the positions of the therapy delivery needle 10 and the reference needle 20, a vector representing relative coordinates of the tumor 4 with respect to the reference positioning needle 20, a deformation parameter of the organ, updated relative coordinates of the tumor 4 with respect to the reference needle 20, relative positions of a therapy delivery needle 10, etc.

In one or more embodiments, the processing module 54 of the device 50 may be configured to determine one or more of: a vector representing relative coordinates of the tumor 4 with respect to the reference needle 20 based on the relative position of the tumor 4 with respect to the reference needle 20 which is determined according to the medical image; updated relative coordinates of the tumor 4 with respect to the reference needle 20 according to the deformation parameter of the organ; and relative positions of a therapy delivery needle 10 relatively to the reference needle 20.

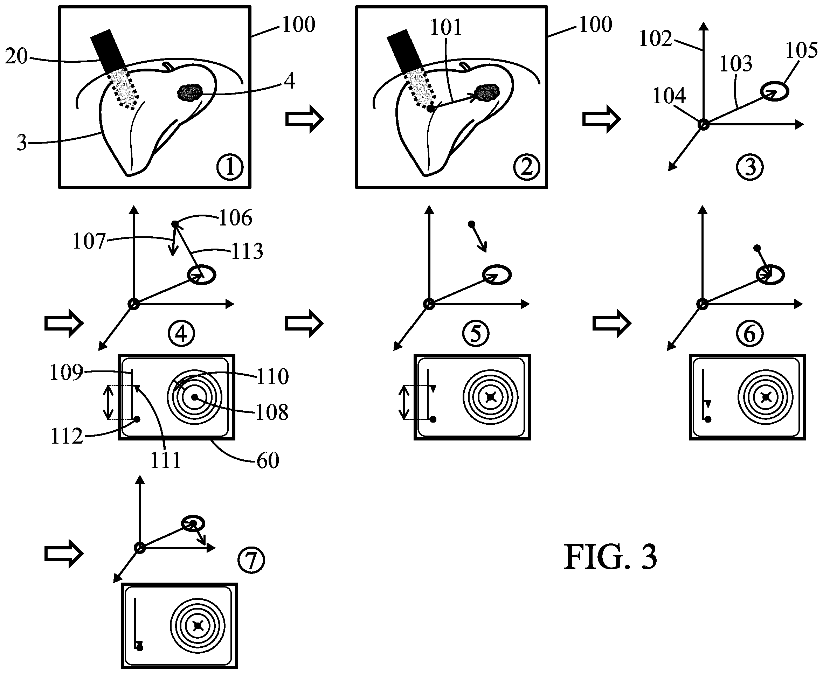

Figure 3 illustrates the determination of a relative position of a therapy delivery instrument relatively to a target region in accordance with one or more embodiments.

The schematic representation number ① on Figure 3 represents a medical image 100 received from a MI unit 40, which is used to detect a reference needle 20 and a tumor 4.

In one or more embodiments, a processing module of the proposed device (such as, for example, the processing module 54 of the exemplary device 50 of Figs. 1 and 2) may be configured to determine the relative position of the tumor 4 with respect to the reference needle 20 based on the image 100.

As shown on the schematic representation number ② of Fig. 3, this relative position may in some embodiments be expressed by a vector V_{I} 101. The device 50 may further be configured to, in addition to determining the vector V_{I} 101, compute other variables including but not limited to: distance to target, approaching angle, target translation, image scale and cross-section, or any other that may be surrogated by the vector V_{I} 101.

In some embodiments, the processing module 54 may further be configured to compute, based on data comprising the vector V_{I} 101, the image scale, and the position of the section, in the coordinate system 102 relative coordinates 105 of the tumors 4 with respect to the reference needle 20.

For example, in some embodiments, the processing module 54 may be configured to transform the vector V_{I} 101 represented according to the image 100 in a vector Vc 103 in the coordinate system 102 in which the different items (the therapy delivery needle 10 and the reference needle 20) tracked by the Spo 31 or by the robot 6 are (or will be) represented, as presented on the schematic representation number ③ on Fig. 3. For example, the vector V_{C} 103 obtained in this coordinate system may start from a point 104 representing the reference needle 20. The tip end of the vector V_{C} 103 may indicate a region 105 representing the tumor 4. The tip end of the vector V_{C} 103 may for example represent the center of the region 105. The coordinate system 102 may be a three-dimensional coordinate system, in which case the tracking of a point may be performed in three dimensions.

Once the Spo 31 or the robot 6 starts tracking the therapy delivery needle 10, the tip end of the therapy delivery needle 10 may be represented by the dot 106 and the direction of the therapy delivery needle 10 may be represented by the direction 107 as presented on the schematic representation number ④ of figure 3. As it can be seen the therapy delivery needle 20 is not heading towards the tumors 4 on the schematic representation number ④. The orientation of the therapy delivery needle 20 may therefore be adapted or updated.

In some embodiments, the device 50 may be configured to inform an operator through the screen 60 by displaying for example a target 108 and a proximity gauge 109.

In cases where the cross 110 is not at the center of the target 108, it may be determined that the orientation of the therapy delivery needle 20 is not aligned with the direction formed by the dot 106 to the center of the tumor 4 (represented by the vector 113). In cases where the cross 110 is at the center of the target 108, it may be determined that the orientation of the therapy delivery needle 20 is aligned with the direction formed by the dot 106 to the center of the tumor 4 as represented on the schematic representation number ⑤ of figure 3.

The distance between the triangle 111 and the dot 112 on the proximity gauge 109 may represent the distance to go between the tip end of the therapy delivery needle 10 and the tumor 4, as it can be seen on the schematic representation numbers ⑤, ⑥ and ⑦.

Other ways of informing the operator may be used for example based on sound indications or vibrations.

The center of the coordinate system 102 is fixed to the reference needle 20 represented by the dot 104. Therefore, in situations where the organ 3 moves, the reference needle 20 and the tumor move approximately in the same manner as the organ 3, which therefore makes it possible to track the position of the tumor 4 by knowing the position of the reference needle 20 and the vector Vc 103. The movement of the organ 3 can for example result from breathing of the patient 1 or evolution of blood pressure resulting from heartbeat. During the movement of the organ 3, the vector 103 stays approximately the same. However, the vector V_{C} 103 may be updated according to a deformation parameter which can be used to determine how the organ 3 stretches and compresses (even slightly) during these movements. In some embodiments, this update of the vector V_{C} 103 may be performed each time the relative position of the therapy delivery needle 10 is determined (for example each time a new position of the reference needle 20 is received by the device 50).

In embodiments using a medical robot 6, the device 50 may be configured to transmit instructions to the robot 6 to orient the therapy delivery needle 10 in the direction of the tumor 4 and to insert more the therapy delivery needle 10. In that case there is no need to implement the screen 60.

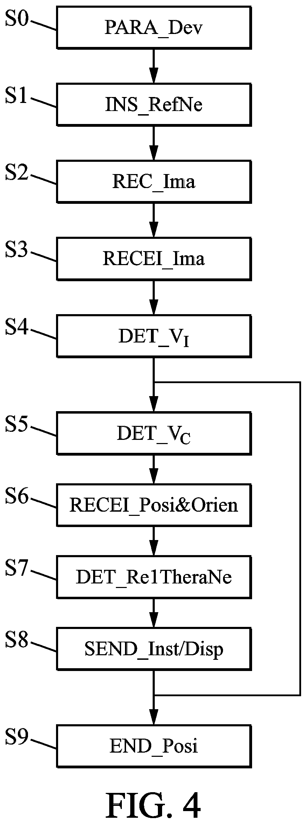

Figure 4 illustrates a flowchart representing an implementation of a system in accordance with one or more embodiments.

As shown on Fig. 4, the device 50 may first be parametrized or initialized (S0). For example, the spatial positioning technique and the medical imaging technique may be configured in the device. According to these techniques the data received from the MI unit 40 and from the Spo 31 or the medical robot 6 can be interpreted by the device 50. The device may also be configured with a coordinate system. For example, the center point of the coordinate system may be chosen collocated with an item in the operating room (such as, for example, the reference needle 20 as shown in figure 3). The device may also be configured with a deformation parameter. Depending on the embodiment, the deformation parameter may be defined based on medical criteria such as the age of the patient, image features of the organ (shape of the organ, density of the organ, etc.), etc., or based on the resistance of the organ to the insertion of the reference needle 20.

The reference needle 20 is then inserted (S1) by the operator 2 or the (medical) robot 6 into the organ.

Once the reference needle 20 is inserted, the resistance of the organ to the insertion of the reference needle 20 may be computed to determine a value of the deformation parameter.

In some embodiments, the MI 40 may also implement (S2) the technique for detecting the region 4 to be treated and the reference needle 20. The MI 40 may produce data based on the implemented technique, for example, a medical image of the organ 3. In some embodiments, the operator 2 may determine based on the data produced by the MI 40 the region to operate 4. In other embodiments, the target region may be determined prior to performing the operation procedure. The reference needle 20 and the region 4 may be detected by the operator 2 or may be detected automatically for example relying on radiopacity or contrast, on the shapes of the region 4 and of the reference needle 20.

In some embodiments, the device 50 may receive (S3) from the MI unit 40 via its imaging interface (INT_MI) 52 data related to the medical image 100. This data may comprise data corresponding to the image 100, data corresponding to the image scale and data corresponding to the section represented by the image 100.

In some embodiments, the processing module 54 may determine (S4) the reference needle 20 and the target region 4 on the image 100. The processing module 54 may further determine a relative position of the target region 4 (for example the tumor 4) with respect to the reference needle 20 based on the data received, for example the image 100. For example, the processing module 54 may determine the reference needle 20 and the target region 4 on the image 100 based on which the processing module 54 may determine the vector V_{I} 101.

In some embodiments, the processing module 54 may determine (S5) in the coordinate system 102 relative coordinates of the target region 4 with respect to the reference needle 20. For that, based on the vector V_{I} 101 determined (S4) and the data corresponding to the image scale and data corresponding to the section represented by the image 100, the processing module 54 determines the vector Vc 103. Since the reference needle 20 is represented (by the spot 104) in the coordinate system 102 as its reference center, the vector V_{C} 103 is represented as starting from spot 104. The tip end of the vector Vc 103 thus represents the coordinates of the target region 4 represented in the coordinate system by the region 105.

The vector Vc 103 is constant, which gives a good approximation of the position of the tumor 4. However, the Vc 103 can be updated according to the deformation parameter each time positions and/or orientations of the reference needle 20 are received from the Spo 31 or from the medical robot 6.

In some embodiments, the device 50 may receive (S6) via its spatial positioning interface (INT_Spo) 51 or the robot interface (INT_ROB) 56 the positions and/or orientations determined respectively by the Spo 31 or by the medical robot 6 of the reference needle 20 and the therapy delivery needle 10.

In some embodiments, the processing module 54 may determine (S7), based on the positions and/or orientations received at step S6, the coordinates and the direction of the therapy delivery needle 20 in the coordinate system 102, which is represented by the spot 106 and the vector 107. The direction 107 may as well be determined based on the evolution of the spot 106 through time. Since in the example of figure 3 the spot 104 (which represents the reference needle 20) is the reference center of the coordinate system 102, the coordinates 106 of the therapy delivery needle 10 corresponds to a relative coordinate of the therapy delivery needle with respect to the reference needle 104. Other reference center may be chosen without affecting the teachings of the subject disclosure.

When the organ 3 moves, the reference needle 20 and the target region 4 move with the organ 3. Thus, the relative position of the target region 4 (for example, the tumor 4) with respect to the reference needle 20 stay slightly the same. Therefore, the relative coordinates 103 of the target region 4 is an accurate estimation of the position of the tumor 3 when the organ 3 moves. Since the reference center of the coordinate system 102 is fixed to the reference needle 20 it is therefore a moving coordinate system 102 regarding a fixe point in the surgical room. The position of the therapy delivery needle 10 depends more on the operator 2 or the medical robot 6 and less on the movements of the organ 3. Therefore, the coordinates 106 and orientation 107 of the therapy delivery needle 10 do not follow the movements of the organ 3. Thus, the coordinates of the therapy delivery needle 10 evolves in this moving coordinate system 102 each time the organ 3 moves.

The coordinates 106 and orientation 107 of the therapy delivery needle 10 are determined based on the positions and orientations received from the Spo 31 or by the medical robot 6 of the therapy delivery needle 10 and based on the position of the reference needle 20 also received from the Spo 31 or by the medical robot 6.

In some embodiments, the processing module 54 may further determine relative coordinates and/or orientation 113 of the therapy delivery needle 10 with respect to the target region 4. This is done by using the coordinates 106 and orientation 107 of the therapy delivery needle 10 and the coordinates 105 of the target region 4. The coordinates of the reference needle 20 are also needed, but in the specific case where the reference center of the coordinate system 102 is fixed to the reference needle 20, these coordinates are the reference center itself and are static in the coordinate system 102.

In some embodiments, the processing module 54 may determine (S8) data to be displayed on the screen 60 based on the relative coordinates and orientation 113 of the therapy delivery needle 10 with respect to the target region 4, and send such data to the screen 60 via the screen interface (INT_SCR) 53. An example of the data to be displayed is shown on Figure 3.

In the case a medical robot 6 is used, no data is displayed, however, instructions (or orders) are sent to the medical robot 6 via the robot interface (INT_ROB) 56. The instructions aim at aligning the vector 107 with vector 113 and then to reduce the distance between item 111 and item 112 by inserting the therapy delivery needle 10, or put in another way to reduce the vector 113.

The acts S5 to S8 may be repeated in a loop until the tip end of the therapy delivery needle 10 reaches the target region 4 (S9).

While the invention has been described with respect to preferred embodiments, those skilled in the art will readily appreciate that various changes and/or modifications can be made to the invention without departing from the spirit or scope of the invention as defined by the appended claims.

Although this invention has been disclosed in the context of certain preferred embodiments, it should be understood that certain advantages, features and aspects of the systems, devices, and methods may be realized in a variety of other embodiments. Additionally, it is contemplated that various aspects and features described herein can be practiced separately, combined together, or substituted for one another, and that a variety of combination and sub-combinations of the features and aspects can be made and still fall within the scope of the invention. Furthermore, the systems and devices described above need not include all of the modules and functions described in the preferred embodiments.

Information and signals described herein can be represented using any of a variety of different technologies and techniques. For example, data, instructions, commands, information, signals, bits, symbols, and chips can be represented by voltages, currents, electromagnetic waves, magnetic fields or particles, optical fields or particles, or any combination thereof.

Depending on the embodiment, certain acts, events, or functions of any of the methods described herein can be performed in a different sequence, may be added, merged, or left out all together (e.g., not all described acts or events are necessary for the practice of the method). Moreover, in certain embodiments, acts or events may be performed concurrently rather than sequentially.

## Claims

1. A device configured for guiding a therapy delivery instrument to a target region of an organ to be treated, comprising:
a processor;
a non-transitory computer-readable medium comprising instructions stored thereon which, when executed by the processor, cause the device to:
- determine a relative position of the target region with respect to a reference positioning tracker inserted into the organ obtained using a technique for detecting a region to be treated and the reference positioning tracker;
- determine in a coordinate system relative coordinates of the target region with respect to the reference positioning tracker, wherein the determination of the relative coordinates is based on the relative position of the target region with respect to the reference positioning tracker;
- determine a relative position of a therapy delivery instrument relatively to the target region based on:
∘ the relative coordinates of the target region with respect to the reference positioning tracker;
∘ current coordinates in the coordinate system of the therapy delivery instrument obtained using a spatial positioning technique; and
∘ current coordinates in the coordinate system of the reference positioning tracker obtained using the spatial positioning technique.

2. The device according to claim 1, wherein the reference positioning tracker is a reference needle.

3. The device according to claim 1 or claim 2, further configured for determining the current coordinates of the reference positioning tracker using a first electromagnetic sensor configured for producing signals in response to an electromagnetic field produced by an electromagnetic generator, wherein the electromagnetic sensor is fixedly coupled to the reference positioning tracker and the current coordinates of the therapy delivery instrument are determined based on signals received from at least a second electromagnetic sensor in response to the electromagnetic field, wherein the second electromagnetic sensor is fixedly coupled to the therapy delivery instrument.

4. The device according to claim 3, further comprising a guiding device configured to guide the therapy delivery instrument, wherein the guiding device comprises a third electromagnetic sensor configured for producing signals in response to the electromagnetic field, wherein the device is further configured for determining the current coordinates of the therapy delivery instrument based on signals received from the third electromagnetic sensor in response to the electromagnetic field.

5. The device according to claim 4, further configured for determining the relative position of the therapy delivery instrument relatively to the target region based on angular coordinates of therapy delivery instrument obtained based on signals received from the third electromagnetic sensor, and on insertion depth value of the therapy delivery instrument relatively to the guiding device obtained based on signals received from the second and third electromagnetic sensors.

6. The device according to claim 3 wherein the second electromagnetic sensor is positioned in a front part of the therapy delivery instrument.

7. The device according to claim 1, further configured for determining the current coordinates of the reference positioning tracker based on information on a current position of an arm of a medical robot and the current coordinates of the therapy delivery instrument are determined based respectively on information on a current position of a second arm of the medical robot.

8. The device according to claim 1, wherein the device is operatively coupled with a robot, and the device is further configured for determining the current coordinates of the reference positioning tracker based on information on a current position of a first arm of the robot, and for determining the current coordinates of the therapy delivery instrument based on information on a current position of an electromagnetic sensor fixedly coupled with the therapy delivery instrument.

9. The device according to claim 1, wherein the device is operatively coupled with a robot, and the device is further configured for determining the current coordinates of the reference positioning tracker based on information of an electromagnetic sensor fixedly coupled with the reference positioning tracker, and for determining the current coordinates of the therapy delivery instrument based respectively on information on a current position of an arm of the medical robot, and on an electromagnetic sensor fixedly coupled with the medical robot.

10. The device according to any one of the preceding claims, the proposed device is further configured to use, as the technique used for detecting a region to be treated, a medical imaging technique, and to use an image obtained by the medical imaging technique for determining the relative position of the target region with respect to a reference positioning tracker inserted into the organ.

11. The device according to any one of the preceding claims, wherein the coordinate system is a frame of reference centered on a point of the reference positioning tracker and with static axes relative to the reference positioning tracker.

12. The device according to any one of the preceding claims, wherein the non-transitory computer-readable medium comprises further instructions stored thereon which, when executed by the processor cause the device to determine a deformation parameter of the organ, wherein determining the relative position of the therapy delivery instrument relatively to the target region is based on the deformation parameter of the organ.

13. The device according to any one of the preceding claims wherein the non-transitory computer-readable medium comprises further instructions stored thereon which, when executed by the processor cause the device to send an order to a robot to reduce a distance between the therapy delivery instrument and the target region based on the relative position of the therapy delivery instrument relatively to the target region.

14. A computer program product comprising code instructions that, when said instructions are run by at least a processor, instruct the processor to perform a method comprising:
- determining a relative position of the target region with respect to a reference positioning tracker inserted into the organ obtained using a technique for detecting a region to be treated and the reference positioning tracker;
- determining in a coordinate system relative coordinates of the target region with respect to the coordinates of the reference positioning tracker, said determination of the relative coordinates being based on the relative position of the target region with respect to the reference positioning tracker;
- determining a relative position of a therapy delivery instrument relatively to the target region based on:
∘ the relative coordinates of the target region with respect to the reference positioning tracker;
∘ current coordinates in the coordinate system of the therapy delivery instrument obtained using a spatial positioning technique; and
∘ current coordinates in the coordinate system of the reference positioning tracker obtained using the spatial positioning technique.
